(19) Europäisches Patentamt
European Patent Office
Office européen des brevets

(11) Publication number: 0 190 464
A2

(12) # EUROPEAN PATENT APPLICATION

(21) Application number: 85116663.7

(22) Date of filing: 31.12.85

(51) Int. Cl.[4]: A 61 K 33/24
A 61 K 9/18, A 61 K 47/00

(30) Priority: 31.12.84 IL 73972

(43) Date of publication of application: 13.08.86 Bulletin 86/33

(84) Designated Contracting States: AT BE CH DE FR GB IT LI LU NL SE

(71) Applicant: YEDA RESEARCH AND DEVELOPMENT COMPANY, LIMITED
P.O. Box 95
Rehovot 76100(IL)

(72) Inventor: Arnon, Ruth
Meonot Wix Weizmann Institute of Science
Rehovot(IL)

(72) Inventor: Wilchek, Meir
Rh. Havoda 3
Rehovot(IL)

(72) Inventor: Schechter, Bilha
Meonot Brazil 1 Weizmann Institute of Science
Rehovot(IL)

(74) Representative: Vossius Vossius Tauchner Heunemann Rauh
Siebertstrasse 4 P.O. Box 86 07 67
D-8000 München 86(DE)

(54) **Novel anti-cancer drugs.**

(57) Described are complexes between a physiologically acceptable macromolecular carrier and an anti-tumor active platinum compound useful as anti-cancer drugs.

EP 0 190 464 A2

VOSSIUS · VOSSIUS · TAUCHNER · HEUNEMANN · RAUH
PATENTANWÄLTE

SIEBERTSTRASSE 4 · 8000 MÜNCHEN 86 · PHONE: (089) 47 40 75
CABLE: BENZOLPATENT MÜNCHEN · TELEX 5-29 453 VOPAT D

Our Ref.: U 275EP December 31, 1985

Field of the invention:

The invention relates to novel anti-cancer drugs based on platinum complexes. There are provided complexes of pharmaceutically active platinum compounds with pharmacologically acceptable high molecular weight compounds. The high molecular weight compounds of choice are of the suitably substituted polysaccharide type or polyamino acids, with the suitable substituents such as, amongst others, amino or carboxymethyl (CM) or hydroxyl groups. The active compound is gradually released from such complex, which has a substantially decreased toxicity.

Background of the invention:

Cis-dichlorodiammine Platinum (II) (cis-Pt), is widely used in the chemotherapy of human cancer. The drug is very toxic and is apt to cause injuries to the bone marrow and intestinal mucosa as well as long-term nephrotoxic effects. The range between effective dosage and toxic ones is very narrow. A large number of other platinum compounds has been evaluated in an effort to provide anti-cancer drugs with increased effectivity and with reduced toxicity. Most of the compounds prepared have proved to be of lesser activity or selectivity.

Cis-Pt, which is a coordination complex, forms complexes with a variety of low molecular weight compounds. Attempts were made to bind such compounds to cis-Pt in order to decrease toxicity while retaining at least part of the anti-tumor activity. Many derivatives have been reported in literature, amongst these there being carriers such as mono- or oligo-peptide esters (about 40 compounds), sulfadiazine, diamino-carboxylate esters and amino-monosaccharides. Most of the thus obtained complexes did not exert an activity approaching that of cis-Pt.

The compound diaquodiammine Platinum (II)-nitrate, (cis-aq) (described in detail in the following), was reported to form metastable complexes with the carboxylic group of glycine, but the compound was not evaluated for biological activity.

Summary of the invention:

There are provided novel anti-cancer drugs which are based on complexes of pharmacologically active platinum compounds with high molecular weight compounds. The novel Pt-complexes with macromolecular carriers are characterized by a substantially reduced toxicity resulting in better tolerance and in a high therapeutic anti-cancer activity. The macromolecular carriers of choice are soluble polysaccharides, such as dextran, sepharose, agar-agar or the like, polyamino acids, such as poly-L-glutamic acid (pGlu), poly-L-aspartic acid (pAsp) and the like. Preferred carriers are dextran-amine (dex-$NH_2$), carboxymethyl dextran (CM-dex)and poly-L-glutamic acid and poly-L-aspartic acid.

Amongst platinum compounds evaluated were cis-Pt, cis-aq and $K_2PtCl_4$.

It was found that cis-Pt forms complexes with proteins, polypeptides, modified polysaccharides and the like which can be used as carriers. The platinum compound-carrier complexes exert their activity either by release of the platinum drug from the carrier, or via degradation (chemical or biological) resulting in a low molecular weight active product. The thus formed complexes have a varying degree of stability, and if cis-Pt is complexed with a carrier A, it will be transposed to carrier B if its affinity to the latter is higher. If B is the target of cis-Pt (such as the target component of a tumor cell, i.e. its DNA),

and if the affinity to B is higher than to A, the cis-Pt-carrier complex will be an effective anti-cancer complex.

Extensive studies were carried out with three Pt derivatives, namely, cis-Pt, which is presumed to complex to a variety of macromolecules via the exchange of one or two of the chlorine atoms, cis-aqua (cis-aq) which can be obtained form cis-Pt as follows:

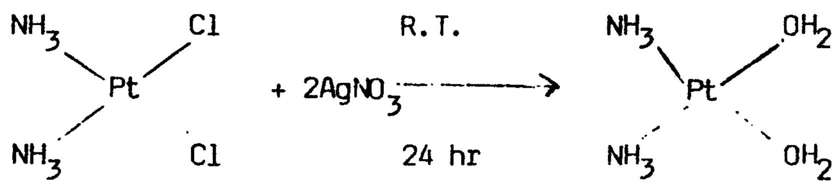


and the platinum salt $K_2PtCl_4$.

Cis-aq is an anti-cancer agent, but its toxicity is so high that it cannot be used in an effective manner in human therapy. Its in vivo toxicity seems to be due to its rapid binding capacity via exchange of the water molecules. $K_2PtCl_4$ as such is 20 to 100 times less active against cancer cells in vitro than cis-Pt or cis-aq and is essentially inactive against tumors in vivo. It was evaluated in a complexed form in order to evaluate whether such complexes have a higher activity.

As will become apparent from the following detailed description, a high degree of binding of cis-Pt, of cis-aq and of $K_2PtCL_4$ to the various macromolecular carriers is attained. Some of the thus obtained complexes were tested in vivo against tumors, and amongst these against F-9 embryonal carcinoma in Balb/c mice. The results show that a high therapeutic activity is attained, with a substantially decreased toxicity of the active Pt compound, and especially of the highly active cis-aq. It is thus possible to make use of cis-aq as drug in the

cancer therapy of mammals, including humans. It was possible to obtain highly effective complexes of cis-Pt or cis-aq with dex-$NH_2$ and also with other carriers such as CM-dextran, pGlu, pAsp. The general toxicity of the cis-Pt compounds was considerably reduced and there was obtained a wide safety margin between the effective dose and toxic the one.

The following detailed description illustrates a number of preferred embodiments of the invention: it ought to be clear that a wide variety of physiologically active macromolecular carriers such as polysaccarides, modified polysaccarides, polyamino acids and the like can be used for the formation of the macromolecular carrier-Pt complexes. These have generally a molecular weight in the 10,000 to 60,000 range. The novel complexes can also be bound to specific antibodies in order to attain a specific targeting to tumor tissues.

Preparation of Macromolecular Carriers

(a) Dextran-$NH_2$ (dex-$NH_2$)

Dex T-10 (Mr-10,000) or T-40 (Mr-40,000) (2.5 g) was dissolved in a 100 ml solution of 0.15 M $NaIO_4$ in double distilled water (DDW). The reaction mixture was kept overnight at room temperature (RT) in the dark and was then dialyzed extensively against DDW (5 x 7 liter). The product was then lyophilyzed, yielding 1.7 g oxidized dex. The oxidized dex, dissolved in 50 ml DDW, was mixed with a solution of 2.4 ml ethylendiamine (EDA) in 50 ml DDW, adjusted to pH 9 with 6N HCl. The reaction mixture was kept at $4^oC$ for 2 hr. The Schiff bases thus formed were then reduced by the addition of $NaCNBH_3$, after adjusting the solution's pH to 7.5. The reaction mixture was kept for 24 hr at $4^oC$, dialyzed as above and lyophilyzed, yielding 600-800 mg of

dex-$NH_2$. The degree of substitution determined by the evaluations of free-$NH_2$ groups (using the trinitrobenzensulfonic acid - TNBS - reagent) was dependent upon the amount of $NaCNBH_3$ added - thus obtaining 8-10 mole-$NH_2$/dex 10,000 and 20-23 mole-$NH_2$/dex 10,000 with 7 and 15 mmole reducing agent, respectively. Some of the EDA was probably bound in the form of $-NH-CH_2-CH_2-NH-$, thus forming inter or intrachain cross links between adjacent -CHO groups. Such groups are unidentified by the TNBS. Since the degree of Pt binding to dex-$NH_2$ was found to be higher than the degree of $-NH_2$ substitution, some cross linked EDA is probably attached to the dex, and participates in Pt complexing. Low yields of dex-$NH_2$ were obtained with both dex T-10 and dex T-40, thus indicating breakage of the dex chains during the reaction.

(b) Carboxymethyl-dextran (CM-Dex)

$-CH_2COOH$ (CM) groups were attached to dextran using sodium hydroxide and chloroacetic acid at a substitution rate of 1 CM/2-3 glucose residues (Soluble Macromolecules as Carriers for Daunorubinin, Hurwitz, E., Wilchek, M., Pitha, J., J. Appl. Biochem. 2, 25-35, (1980)).

(c) Poly-L-glutamic acid (pGlu) and poly-L-aspartic acid (pAsp)- are available at a variety of molecular weights (50, 100, 140, 450 mer). Two (pGlu) preparations (Mr 13,000 and Mr 58,000) were used in the experiments described below. A p(Asp) preparation of Mr of about 12,000 was used.

Platinum complexes

Using the 3 Pt-derivatives and the 4 carrier molecules mentioned above, twelve Pt-carrier combinations were obtained:

| Carrier / Pt-compound | $Dex-NH_2$ | CM-DEX | pGlu | pAsp |
|---|---|---|---|---|
| Cis-Pt | $Cis-Pt-Dex-NH_2$ | Cis-Pt-CM-Dex | Cis-Pt-pGlu | Cis-Pt-pAsp |
| Cis-aq | $Cis-aq-Dex-NH_2$ | Cis-aq-CM-Dex | Cis-aq-pGlu | Cis-aq-pAsp |
| $K_2PtCl_4$ | $K_2PtCl_4-Dex-NH_2$ | $K_2PtCl_4$-CM-Dex | $K_2PtCl_4$-pGlu | $K_2PtCl_4$-pAsp |

Determination of Pt-derivatives binding

The 3 Pt-derivatives dissolved in DDW were routinely quantitated using the reagent Ortho (1,2) phenylenediamine (OPDA) (dissolved in DMF). Each of these compounds interacts with OPDA at $100^oC$ to form a blue color reaction with maximum absorbance at 703 nm. Fig. 1 illustrates the absorption patterns of the Pt-derivatives after 1 and 7 minutes reaction with OPDA; After 7, (or more), minutes reaction with OPDA, the absorption patterns are linear and those of cis-aq and cis-Pt are identical and only slightly lower than that of $K_2PtCl_4$. Binding of Pt-derivatives to the carriers was calculated either from the determination of unbound Pt in total reaction mixtures ($dex-NH_2$ complexes) or of bound Pt in dialyzed complexes (CM-dex, pGlu and pAsp complexes). Pt that is complexed to the carrier with low affinity interacts with OPDA under the conditions described above. Pt determinaton was performed with reference to standard solutions.

Evaluation of Anti-Tumor Activity In-Vitro

Inhibition of DNA synthesis by the free platinum compounds of their complexes was determined as follows. Tumor cells ($2-5 \times 10^4/0.1$ ml) suspended in their suitable grwoth media were dispersed into wells of microtiter plates. The Platinum compounds or complexes were added

to the cells in 25 ml aliquots. The plates were incubated for 21 hr at 37°C in a humidified atmosphere of 5% $CO_2$ in air. At that time 10 µl containing 0.5 µci of [$^3$H]-methylthymidine were added to each well and the plates were incubated for 3 hr as above. The reaction was terminated using a Titerteck cell harvester.

Experiments in vivo

In vivo experiments were performed with F-9 murine embryonal carcinoma cells which were maintained in-vitro in DMEM supplemented with 10% fetal calf serum, glutamine and antibiotics, in gelatine (0.1%) coated culture flasks. In-vivo experiments were performed in female Balb/c mice at the age of 2-3 months.

Pt-carrier Macromolecular Complexes

EXAMPLE 1

Dex-$NH_2$ complexes

Pt-derivatives were incubated with various amounts of dex-$NH_2$, all dissolved in DDW. Table 1 describes the binding efficacy, in percentage of total Pt, as a function of Pt: dex-$NH_2$(10,000 MW) ratios, temperature, EDA substitution and time of incubation. As is shown, the extent of binding is usually higher at 37°C than at RT, and increases with time of incubation. Cis-aq often binds better than Cis-pt, whereas $K_2PtCl_4$ seems to bind better than both. Percentage of binding increases with decreased Pt: dex-$NH_2$ ratio, that is, only 25% of the Pt derivative is bound at a ratio of 40 mole Pt: mole dex-$NH_2$, 50% at a ratio of 20:1, 80% at 10:1 and 99-100% at 5:1. The high affinity of $K_2PtCl_4$ to dex-$NH_2$ and the insolubility of its complexes at high $K_2PtCl_4$: dex-$NH_2$ ratios were presumably due to the participation of both potassium and chlorine atoms in the formation of the complex.

Pt complexes used in the *in vitro* and *in vivo* experiments were prepared under conditions of maximal binding, that is, 98-100% binding of the free drugs. *In vivo* experiments were performed with cis-Pt and cis-aq complexes. Fig. 2 illustrates the inhibition of DNA synthesis of 38C-13 tumor cells (cells were incubated with drugs or drug complexes for 21 hr at 37°C, followed by a 3 hr [$^3$H]-methylthymidine pulse) by the three Pt-derivatives: $K_2PtCl_4$ was 20 fold less inhibitory than cis-Pt and cis-aq (50% inhibition was obtained by 20 x $10^{-6}$M $K_2PtCl_4$ and by 0.8-1 x $10^{-6}$M cis-Pt or cis-aq). Cis-Pt and cis-aq complexes of dex-$NH_2$ were tested and found to be active *in vitro* against 5 murine tumors (EL-4, 38-C13, L1210, RDM and F-9) and 2 human derived tumors (Hela and Osteosarcoma). The dex-$NH_2$ complex with $K_2PtCl_4$ was not inhibitory while cis-Pt and cis-aq complexes inhibited DNA synthesis being only 1.7 and 1.2 less inhibitory than the respective free drugs. This activity was lost, however upon exhaustive dialysis.

*In vivo* experiments with F-9 tumor cells were carried out with cis-Pt and cis-aq complexes of dex-$NH_2$ in comparison to the free drugs (Table 2). In experiment I, 300,000 tumor cells were inoculated intraperitoneally (I.P.) into Balb/c mice. Control mice that did not received further treatment died of the tumor at 34-67 days after tumor injection. Four out of 5 tumor bearing mice (TBM) that received 100 ug cis-Pt. I.P. 1 day after tumor inoculation died of drug toxicity and 1/5 died of the tumor 54 days after receiving the tumor cells. TBM treated with three doses of 150 µg cis-Pt complexed to dex-$NH_2$ (on days 1,6 and 12 after the tumor) did not die of toxicity and did not develop any tumors. Out of 5 TBM treated with 50 µg cis-aq, 1 died of drug

toxicity, 3 died of the tumor and 1 survived. Four out of 5 mice treated twice with 150 µg cis-aq complexed to dex-$NH_2$ survived whereas 1/5 died of toxicity.

In experiment II, mice were given a higher tumor load (600,000 cell I.P.) and treatment began 2 days after tumor inoculation. Cis-Pt (60 µg) was non-toxic and protective against the tumor in 4/5 mice, whereas 60 µg cis-aq were toxic in 3/5 and non-protective against the tumor in 2/5 mice. Dex-$NH_2$ complexes of cis-Pt and cis-aq (100 µg x 3) administered at days 2,12 and 24 did not result in death due to toxicity and were each protective against the tumor in 5/5 mice. Decrease in mice whole body weight due to the toxic effects of the drugs was noticed with the free drugs and with 150 µg x 3 but not with 100 µg x 3 complexed drugs.

Thus, the complexed drugs are active <u>in vivo</u> and are much better tolerated than the respective free drugs by the tumor bearing mice.

<u>Example 2</u>

<u>CM-Dex complexes</u>

Cis-Pt and cis-aq form biologically active complexes with dextran (dex) subsititued with carboxymethyl (CM) groups at an average substitution ratio of 1 mole CM per 2-3 mole glucose units of dextran. The complexes formed are different from those of dex-$NH_2$. With CM-dex, most of the Pt or all of it is reversibly bound to the carrier, i.e. it can be transposed to another acceptor of higher affinity to the Pt, such as OPDA, if introduced into the system. The complexes were formed by reacting each of the two Platinum (II) derivatives with carboxymethyl-dextran (CM-dex) at room temperature (RT) or at 37$^{o}$C in an aqueous solution. The complexes were then dialyzed to remove unbound Pt and their remaining Pt content was determined by a reaction with OPDA

for 10 min. The complexing rate increased with temperature, Platinum (II):CM-dex ratio in the reaction mixture (Table 3) and with time of reaction up to around 1 mole Platinum (II) per 1-3 mole CM group after 24 hr at 37$^0$C. Experiments were performed with two CM-dex preparations, one derived from dex T-10 (Mr-10,000) and the other from dex-40 (Mr-40,000). Soluble cis-Pt and cis-aq complexes formed with CM-dex T-10 and CM-dex T-40 carried up to 14 mole or 60 mole of the Platinum (II) compounds per 1 mole CM-dex, respectively. Reactivity of cis-aq with CM-dex was higher than that of cis-Pt. NaCl interfered with complex formation, but did not cause dissociation of already formed complexes. The complexes were stable upon storage at 4$^0$C and their complexed Platinum (II) content remained unchanged. However, the binding of cis-Pt and cis-aq to CM-dex is reversible and the drugs can be transposed to other acceptors of higher affinity to Platinum(II) for example, DNA.

The complexes were active against tumor cells in vitro and in vivo. The activity in vitro was only somewhat lower than that of the free drugs. (Table 4).

The in vivo chemotherapeutic effect of the different complexes in comparison with free drugs was evaluated by the suppression of F9 embryonal carcinoma in Balb/c mice. The experiments were performed with cis- Pt and cis-aq complexes of CM-dex T-10 and CM-dex T-40. As is shown in Table 5, the F9 tumor was generally, but not always susceptible to free cis-Pt or cis-aq treatment when given at the highest non-toxic doses. The free drugs were effective at a narrow dose range of 50-60 µg/mouse for cis-Pt and 40 µg/mouse of cis-aq. Lower doses were sub-effective whereas higher doses resulted in at least partial death due to toxicity, and the mice that survived the toxic effects of the

drugs generally died of the tumor. The CM-dex complexes were less toxic, although reduction in toxicity was accompanied by a decrease in drug effectivity. There was a difference in activity between the two types of CM-dex complexes differing in molecular size. The complexes of the drugs with CM-dex T-40 are similar in their properties to the free drugs, but their efficacy was higher since the effective doses were tolerated by the mice and led to 100% long term survival. The cis-Pt complex was effective at 100 µg/mouse but was toxic at higher doses whereas the cis-aq, that was more toxic than cis-Pt was effective at a higher dose range when complexed to CM-dex (100-150 µg/drug/mouse). The complexes of cis-Pt and cis-aq with CM-dex T-10 were less effective than the respective CM-dex T-40 complexes, but they were of much lower toxicity and showed essentially no toxic effects towards the mice, even at a dose of 200 µg/mouse. Further more, as shown in the lower part of the Table, the efficacy of these macromolecular complexes could be dramatically increased if administered at a multi-dose schedule. Due to its low toxicity, the mice could tolerate three repeated injections of 100-150 µg/mouse resulting in 100% survival without significant toxic manifestations such as loss of body weight.

Repeated injections of the CM-dex T-40 complexes were restricted due to toxicity and could be given either at lower doses or at larger time intervals as indicated in Table 5. The free durgs, when administered at three repeated injections of the highest non-toxic dose, showed high toxicity in tumor-free mice and led to over 50% mortality.

EXAMPLE 3

pGlu-complexes

The two Platinum (II) derivatives were tested for their ability to form reversible complexes with pGlu (two preparations were used of Mr-13,000 and Mr-58,000) and pAsp (Mr.-12,000). Cis-Pt and cis-aq were each mixed with the polymers at a ratio of one mole drug per 10 mole carboxylic groups of the polymers. The reaction mixtures were kept at 37$^{o}$C for 1 to 24 hr, after which they were dialysed to remove unbound drug. Their Platinum(II) content was determined using the OPDA reagent.

In general, after interaction of 24 hr, 50-60% of the initial Platinum (II) input reacted with OPDA, both before and after dialysis, indicating that this amount was bound to the polymers in a reversible manner (Table 6). At Pt:carboxyl group molar ratios of 1:5, 1:10 or 1:20 the complexes were soluble. Increasing the Pt ratio to 1:2.5 resulted in precipitation of the complexes. There were no significant differences between the types of pGlu (Mr-13,000 and Mr-58,000) used in this study. Similar results were obtained with the pAsp complexes. The stability of the complexes was demonstrated: no major changes such as increase in proportion of the high affinity bound Platinum (II) took place upon 3 months storage at 4$^{o}$C.

The complexes of cis-Pt and cis-aq with pGlu and pAsp exerted in vitro activity on various tumor cells albeit somewhat lower than that of the free drugs. The chemotherapeutic effect of cis-Pt and cis-aq complexes of pGlu (Mr-58,000) and (Mr 13,000) was tested in vivo in comparison to the free drugs (Table 7). Balb/c mice were inoculated with the F9 tumor cells and one day later the free or complexed drugs were injected via the same route. Free cis-Pt or cis-aq, when given at optimal doses,

resulted in an average of 60-80% survival, but at a slightly higher dose they were at least partially toxic. The complexes at a (releasable) drug dose of 200 ug drug/mouse were as effective or even more effective than the free drugs with no significant differences between the activity of the low and high molecular weight complexes. the most important feature demonstrated is that the toxicity of the complexed drugs was drastically reduced. Thus, the cis-Pt or cis-aq complexes of pGlu (Mr-13,000), that were highly effective in preventing tumor growth at 200 µg/mouse were completely non-toxic even at a level of 800-900 µg/mouse.

Toxicity in IRC mice

Preliminary toxicity experiments with CM-dex and dex-$NH_2$ complexes were performed to compare the toxicity of the free drugs and the complexes in tumor-free ICR mice. Pairs of mice were given a single I.V. injection of each of the drugs or drug complexes at a wide concentration range. Death of mice due to toxicity occurred between 1 to 7 days after injection. The results (Table 8) indicate the maximal non-toxic (none of the mice died) and the minimal toxic dose (death of both mice). As shown both drugs in the free state showed relatively high toxicity (10-14 mg/kg) cis-aq being more toxic than cis-Pt. The CM-dex T-40 of both drugs were also toxic although somewhat less than the free drugs, with no significant difference between the cis-Pt and cis-aq complexes. The CM-dex T-10 complexes were of low toxicity and even the highest doses that were tested namely, 48 and 60 mg/kg respectively, did not lead to any mortality. The dex-$NH_2$ were also considerably less toxic than the free drugs.

The cis-Pt and cis-aq complexes described here present a new class of anti-tumor agents. Active Pt carrier complexes are advantageous over the free drugs since they regulate drug distribution in the body, facilitate slow and continuous drug release and exert much lower toxicity. Furthermore, such complexes are suitable for the attachment to anti-tumor antibodies to form active drug-carrier antibody conjugates for use in either radioimmunotherapy or immunochemotherapy.

<u>Legend to the Figures:</u>

Fig. 1 Spectrophotometric determination of cis-Pt, cis-aq and $K_2PrCl_4$ with o-phenylenediamine (OPDA) - 1 and 7 min. reaction.

Fig. 2 Inhibition of DNA synthesis of 38C-13 leukemia cells by cis-Pt (●) cis-aq (x) and $K_2PtCl_4$ (o).

Table 1: Binding of cis-Pt, cis-aq, and $K_2PtCl_4$ to $Dex-NH_2$[a]

| Pt/dex T-10 | hr | temp | cis-Pt | | | | cis aq | | | | $K_2PtCl_4$ | | | |
|---|---|---|---|---|---|---|---|---|---|---|---|---|---|---|
| | | | % binding at Pt:dex-EA ratio | | | | | | | | | | | |
| | | | 5:1 | 10:1 | 20:1 | 40:1 | 5:1 | 10:1 | 20:1 | 40:1 | 5:1 | 10:1 | 20:1[b] | 40:1[b] |
| $Dex-NH_2$ | 1 | RT | 75 | 34 | 25 | 14 | 93 | 60 | 35 | 12 | 93 | 83 | 64 | 38 |
| 20 $NH_2$/T-10 | 5 | | 82 | 47 | 29 | 30 | 92 | 68 | 45 | 23 | 98 | 97 | 89 | 59 |
| | 24 | | 99 | 70 | 56 | 27 | 97 | 78 | 64 | 52 | 99 | 98 | 94 | 79 |
| | 1 | 37°C | 78 | 45 | 23 | 9 | 93 | 65 | 32 | 22 | 97 | 91 | 77 | 45 |
| | 5 | | 89 | 52 | 25 | 9 | 94 | 72 | 48 | 33 | 99 | 97 | 91 | 72 |
| | 24 | | 99 | 79 | 49 | 25 | 99 | 88 | 64 | 52 | 100 | 97 | 94 | 83 |

a. Pt derivatives (at a concentration of 6.6 µmole/ml) were incubated with various concentrations of $dex-NH_2$ (all in DDW) at a ratio of Pt to dex T-10 (mw-10000) as noted above. Incubation proceeded for 1, 5, 24, hr at either room temperature or at 37°C. A preparation of dex was used: $dex-NH_2$ which contained 20 $NH_2$/T-20.

b. Precipitation accoured at these rations of $K_2PtCl_4$ to $dex-NH_2$ which increased with time of incubation.

Table 2: In vivo effects of cis-Pt and cis-aq complexes of dex-$NH_2$ against F-9 embryonal carcinoma in Balb/c mice[a].

| treatment | day of treatment | # mice | day of death | LTS | change in weight (%) | | | |
|---|---|---|---|---|---|---|---|---|
| | | | | | day-6 | 12 | 38 | |
| I. | | | | | | | | |
| Cis-Pt 100 μg | 1 | 5 | 4,12,12,12,54 | - | -21 | | | |
| Cis-aq 50ug | 1 | 5 | 12,38,42,57 | 1 | -18 | - 9 | | |
| Cis-Pt-dex-$NH_2$ 150 μgx3 | 1,6,12 | 5 | | 5 | -16 | -12 | -7 | |
| Cis-aq-dex-$NH_2$ 150 μgx2 | 1,12 | 5 | 1 | 4 | -20 | - 4 | +3 | |
| Control | | 6 | 34,36,42,45,54,67 | - | - 1 | 0 | +9 | |
| II | | | | | day-5 | 10 | 19 | 29 |
| Cis-Pt 60 μg | 2 | 5 | 33 | 4 | - 6 | - 2 | +7 | +8 |
| Cis-aq 60 μg | 2 | 5 | 7,7,9,33,43 | - | -13 | - 5 | | |
| Cis-Pt-dex-$NH_2$ 100x3 | 2,12,24 | 5 | | 5 | - 1 | + 3 | +6 | +7 |
| Cis-aq-dex-$NH_2$ 100x3 | 2,12,24 | 5 | | 5 | - 1 | + 4 | +5 | +5 |
| Control | | 7 | 21,23,26,38,38,42,45 | - | + 2 | + 2 | -1 | |

a. mice were inoculated I.P. with tumor cells ($3 \times 10^5$ in Exp I and $6 \times 10^5$ in Exp II). Drugs were injected I.P. as indicated.

b. Long term survivors.

c. Mean change in whole body weight of the surviving mice at day after tumor inoculation.

d. Underlined numbers indicate death due to toxicity.

Table 3: Binding of cis-Pt and cis-aq to CM-dex T-10 and CM-dex T-40[a]

| CM-dex T-10 | | | | CM-dex T-40 | | | |
|---|---|---|---|---|---|---|---|
| Molar ratio[b] | dialysis | cis-Pt | cis-aq | Molar ratio[b] | dialysis | cis-Pt | cis-aq |
| 5:1 | - | 91[c] | 97 | 20:1 | - | 98 | 98 |
| | + | 92 | 96 | | + | 97 | 95 |
| 10:1 | - | 90 | 87 | 40:1 | - | 89 | 83 |
| | + | 75 | 70 | | + | 75 | 76 |
| 20:1 | - | 80 | 77 | 80:1 | - | 85 | 86 |
| | + | 55 | 68 | | + | 58 | 75 |
| 40:1[d] | - | 80 | 87 | 160:1 | - | 85 | 86 |
| | + | 46 | 49 | | + | 46 | 51 |

[a] Cis-DDP and cis-aq were each reacted with CM-dex for 17 h at 37°C after which half of each reaction mixture was dialyzed. The platinum (II) content was determined by a 10 min OPDA reaction.

[b] Platinum (II):CM-dex molar ration.

[c] Percent platinum (II) reacting with OPDA.

[d] At these ratios the complexes were insoluble.

Table 4: *In vitro* cytotoxic activity of cis-Pt and cis-aq complexes of CM-dex[a]

| Tumor | CM-dex | Inhibition[b] Free cis-Pt | Complexed cis-PtP | Free cis-aq | Complexed cis-aq |
|---|---|---|---|---|---|
| F9 embryonal carcinoma | T-10 | 0.5 | 0.55 | 0.5 | 0.8 |
| | T-40 | 0.5 | 0.35 | 0.5 | 0.7 |
| 38c-13 lymphoma | T-10 | 0.7 | 1.7 | 0.8 | 1.8 |
| | T-40 | 0.7 | 1.7 | 0.8 | 2.0 |
| L1210 lymphoma | T-40 | 0.7 | 1.4 | 0.82 | 0.9 |
| RDM lymphoma | T-40 | 0.52 | 1.5 | 1.25 | 3.1 |
| EL-4 lymphoma | T-40 | 0.7 | 1.6 | 1.3 | 2.5 |
| Hela cells | T-40 | 0.7 | 1.2 | 1.5 | 0.97 |
| Human osteogenic sarcoma | T-40 | 2.5 | 6.0 | 5.5 | 9.0 |

[a] The tumor cells were incubated with uncomplexed and complexed drugs for 24 h. A pulse of $^3$H-methylthymidine was given at the last 3 h of incubation. The decrease in incorporation of [$^3$H]-methylthymidine into DNA of treated cells as compared with untreated cells was taken as a measure of the inhibitory or toxic activity of the drug.

[b] Drug concentration x $10^{-6}$M that is required in order to cause 50% inhibition of incorporation.

Table 5: The chemotherapeutic effect of cis-Pt and cis-aq complexes of CM-dex T-10 and CM-dex T-40[a]

| Treatment (IP) | Drug (μg/mouse) | cis-Pt Toxic death(%) | cis-Pt ILS[b](%) | cis-Pt S[c](%) | cis-aq Toxic death (%) | cis-aq ILS[b](%) | cis-aq S[c](%) |
|---|---|---|---|---|---|---|---|
| Free drug | 30 | - | 65 | 20 | | | |
| | 40 | | | | 0 | 134 | 80 |
| | 50 | | 270 | 70 | 20 | 0 | 20 |
| | 60 | 20 | - | 80 | 60 | 0 | 0 |
| | 100 | 80 | - | 20 | 100 | - | 0 |
| | 150 | 100 | - | 0 | | | |
| Drug-CM-dex T-10 | 50 | 0 | 38 | 0 | 0 | 30 | 20 |
| | 100 | 0 | 87 | 57 | 0 | 78 | 0 |
| | 150 | 0 | 63 | 57 | 0 | 132 | 0 |
| | 200 | 0 | - | 80 | 0 | 70 | 40 |
| Drug-CM-dex T-40 | 50 | 0 | 58 | 50 | 0 | 110 | 20 |
| | 100 | 0 | - | 100 | 0 | - | 100 |
| | 150 | 43 | - | 43 | 0 | - | 100 |
| | 200 | 80 | - | 0 | 40 | - | 60 |
| Drug-CM-dex T-10 | 100x3 | 0 | - | 100 | 0 | - | 100 |
| | 150x3 | 0 | - | 80 | 0 | - | 100 |
| Drug-CM-dex T-40 | 50x2 | 0 | - | 100 | 0 | - | 100 |
| | 100x2 | 0 | - | 100 | 0 | - | 100 |

[a] F9 embryonal carcinoma cells ($4x10^5$/mouse) were inoculated IP into Balb/c mice (5-7 mice/group, mice body weight was 20-22 g). Single treatments were given one day after the tumor. Three repeated treatments of drug CM-dex T-10 were given 1, 5 and 10 days after tumor inoculation, whereas two repeated treatments of drug CM-dex T-40, 50 or 100 μ/mouse were given at days 1 and 5 or 1 and 24, respectively.

[b] ILS = increase in life span, (median survival of treatment - median survival of control divided by the median survival of the control x 100).

[c] S = survival of mice at day 120.

Table 6: Binding of cis-Pt and cis-aq to p-Glu and p-Asp. Effect of reaction time and platinum (II):-COOH ratio.

| Ratio[b] | Reaction time (h) | Dialysis | Platinum (II) reacting with OPDA(%) | | | |
|---|---|---|---|---|---|---|
| | | | p-Glu | | p-Asp | |
| | | | cis-Pt | cis-aq | cis-Pt | cis-aq |
| 1:2.5 | 24 | - | ±[c] | +++ | ++ | +++ |
| 1:5 | 24 | - | 53 | 44 | 65 | 50 |
| | | + | 54 | 47 | 43 | 50 |
| 1:10 | 1 | - | 80 | 51 | 85 | 68 |
| | | + | 0 | 40 | 5 | 33 |
| | 7 | - | 68 | 50 | 78 | 65 |
| | | + | 16 | 42 | 30 | 45 |
| | 24 | - | 60 | 54 | 73 | 65 |
| | | + | 52 | 52 | 62 | 56 |
| 1:20 | 24 | - | 62 | 65 | 70 | 62 |
| | | + | 57 | 56 | 65 | 57 |

[a] Cis-Pt and cis-aq were each mixed with each of the polymers under the conditions indicated in the Table. The reaction mixtures were kept at 37°C for 24 h after which duplicate samples were diluted to the appropriate platinum (II) concentration, one sample was dialysed whereas the other was kept in the cold.

[b] Platinum (II):COOH ratio.

[c] Degree of precipitation.

Table 7: The chemotherapeutic effect of cis-Pt and cis-aq complexes of p-Glu[a]

| | Drug μg/mouse | MDS[b] | ILS[c] | S(%)[d] | Toxic dose μg/mouse |
|---|---|---|---|---|---|
| cis-Pt | 60 | 67 | 200 | 60 | 100 |
| cis-Pt-p-Glu Mr 13000 | 200 | | | 100 | 900 |
| cis-Pt-p-Glu Mr 58000 | 200 | 61 | 170 | 80 | 700-800 |
| cis-aq | 40 | 76 | 240 | 80 | 60 |
| cis-aq-p-Glu Mr 13000 | 200 | 61 | 170 | 80 | 900 |
| cis-ao-p-Clu Mr 58000 | 200 | | | 100 | 500-600 |
| control | - | 22.5 | | | |

[a] Balb/c female mice (3 months old) were inoculated IP with $3x10^5$ F9 cells. One day later the free drugs and drug complexed with injected IP (5 mice/group).

[b] MDS-mean day of survival.

[c] ILS - increase in life span.

[d] S - survival at day 90.

Experiments were terminated after 90 days.

Table 8: Toxicity of cis-Pt, cis-aq and their CM-dex and dex-$NH_2$ complexes [a]

| Treatment | Drug mg/kg Non toxic | Drug mg/kg Toxic |
|---|---|---|
| Cis-Pt | 10 | 14 |
| Cis-Pt CM-dex T-10 | 48 | - |
| Cis-Pt CM-dex T-40 | 14 | 27 |
| Cis-Pt-dex $NH_2$ | 32 | 45 |
| Cis-aq | 4.7 | 9.5 |
| Cis-aq CM-dex T-10 | 60 | - |
| Cis-aq CM-dex T-40 | 16 | 32 |
| Cis-aq dex-$NH_2$ | 30 | 34 |

[a] Pair of ICR mice were given a single IV injection of the drugs or

Claims:

1. A complex between a physiologically acceptable macromolecular carrier and a platinum compound.

2. A complex according to claim 1, wherein the platinum compound is $K_2PtCl_4$, cis-Pt or cis aq.

3. A complex according to claim 1 or 2, wherein the carrier is a polysaccharide or a polyamino acid, optimally substituted by a functional group facilitating the formation of the complex with the platinum compound.

4. A complex according to claim 3, where the substituents are amino, carboxy or hydroxy groups.

5. A complex according to any of claims 1 to 4, wherein the carrier is a polysaccaride selected from dextran, sepharose, agar-agar and functional derivatives of any of these and from polyamino acids.

6. A complex according to any of claims 1 to 5, wherein the carrier is dextran-amine, carboxymethyl dextran, polu-L-glutamic acid or poly-L-aspartic acid.

7. Complexes between polymeric carriers and platinum compounds substantially as hereinbefore described and with reference to any of the Examples.

8. A pharmaceutical composition comprising as active ingredient a therapuetically active quantity of a macromolecular carrier-platinum complex as claimed in any of claims 1 to 7, with a suitable carrier or adjuvant.

9. A pharmaceutical composition as claimed in claim 8, wherein the active ingredient is a complex of cis-Pt with dextran-amine with carboxymethyl-dextran, with poly-L-glutamic acid or with poly-L-aspartic acid.

10. A pharamceutical composition according to claim 8, wherein the active ingredient is a complex of cis-aq with a physiologically acceptable polymeric carrier selected from dextran-amine, carboxymethyl dextran, poly-L-glutamic acid and poly-L-glutamic acid.

11. A pharmaceutical composition as claimed in any of claims 7 to 10 in unit dosage form.

12. A pharmaceutical composition containing as active ingredient a conjugate or complex of a macromolecular carrier and a Pt-complex substantially as hereinbefore described and with reference to any of the Examples.

13. A method for the treatment of tumors which comprises administering to the patient an effective quantity of a composition claimed in any of claims 8 to 12.

14. An anti-cancer drug suitable for covalent linkage to anti-tumor antibodies for the purpose of immunotargeting or immunoimaging.

0190464

1/2

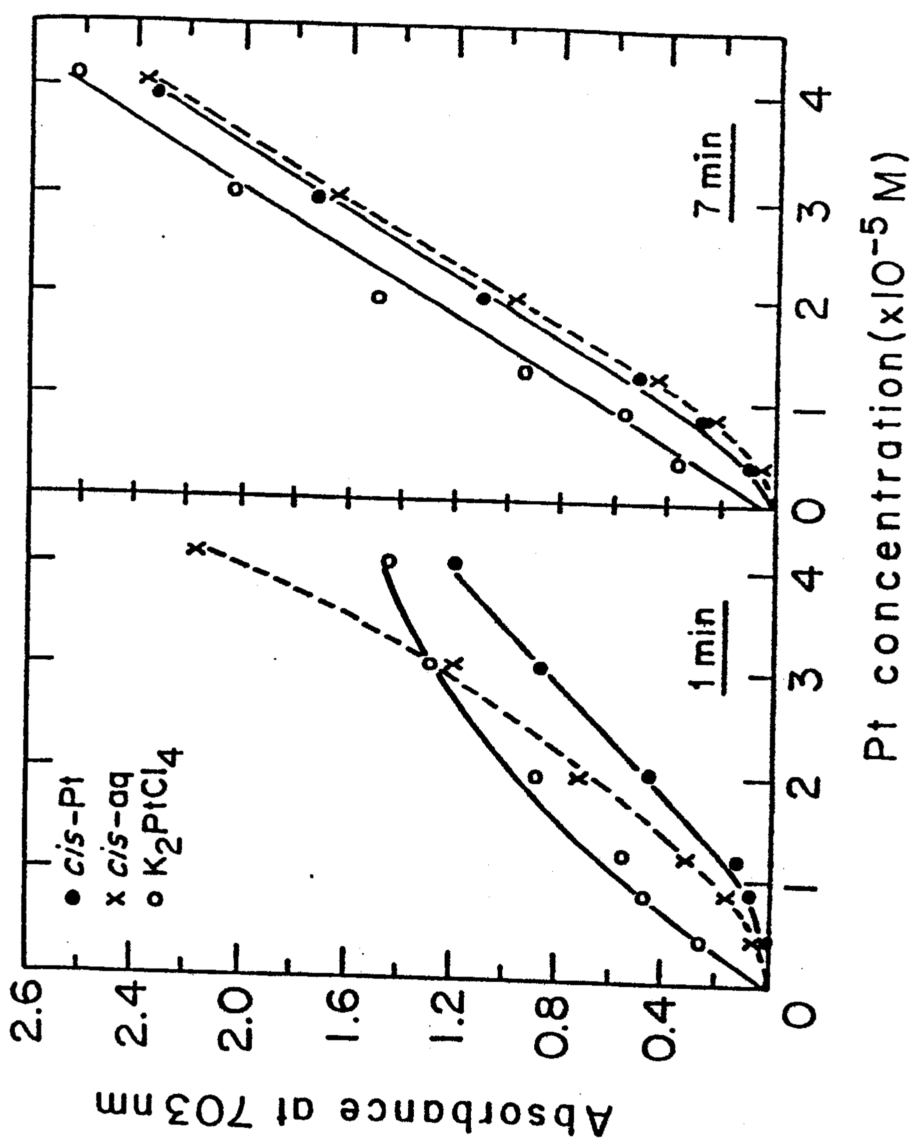


FIG. 1

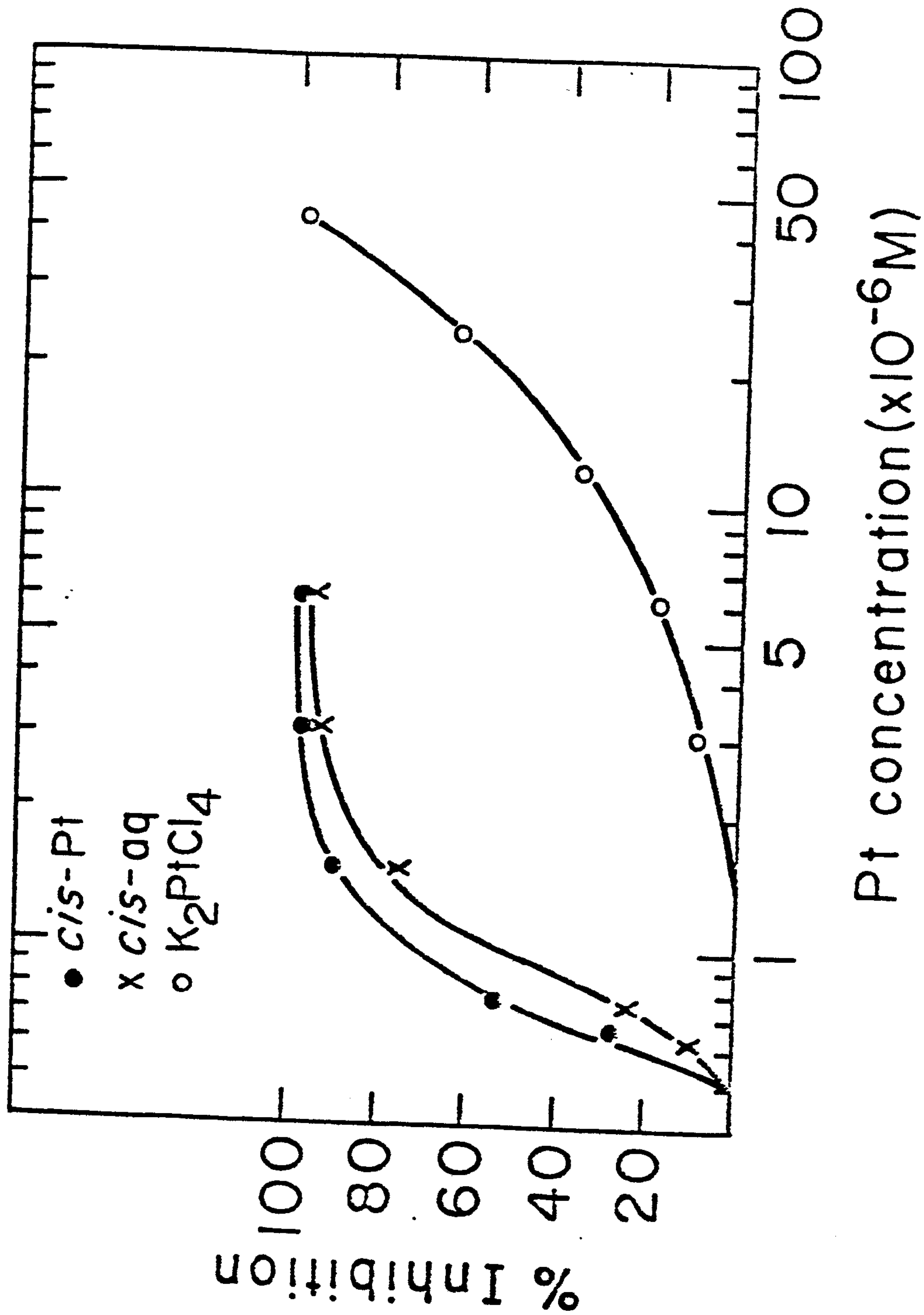

• cis-Pt
x cis-aq
o $K_2PtCl_4$
% Inhibition
100
80
60
40
20
Pt concentration (x10$^{-6}$M)
1
5
10
50
100


Fig. 2